# EUROPÄISCHE PATENTSCHRIFT

(11) **EP 0 906 299 B1**
(45) Veröffentlichungstag und Bekanntmachung des Hinweises auf die Patenterteilung: **04.08.2004**
(21) Anmeldenummer: 97925004.0
(22) Anmeldetag: 28.05.1997
(51) Int. Cl.: C07D 307/08, C07C 41/26, C07C 41/01

(54) **VERFAHREN ZUR HERSTELLUNG VON TETRAHYDROFURAN AUS DIALKOXYBUTENEN**
PROCESS FOR PREPARING TETRAHYDROFURAN FROM DIALKOXYBUTENES
PROCEDE DE PREPARATION DE TETRAHYDROFURANE A PARTIR DE DIALCOXYBUTENENE

(30) Priorität: 05.06.1996 DE 19622497
(43) Veröffentlichungstag der Anmeldung: 07.04.1999
(73) Patentinhaber: BASF AKTIENGESELLSCHAFT, 67056 Ludwigshafen (DE)
(72) Erfinder: FISCHER, Rolf, D-69121 Heidelberg (DE); PINKOS, Rolf, D-67098 Bad Dürkheim (DE); SCHÄFER, Martin, D-67063 Ludwigshafen (DE); HÖHN, Arthur, D-67281 Kirchheim (DE); SCHWAB, Peter, D-67098 Bad Dürkheim (DE)
(86) Internationale Anmeldenummer: PCT/EP1997/002760
(87) Internationale Veröffentlichungsnummer: WO 1997/046546

(56) Entgegenhaltungen:
- EP-A- 0 018 164
- EP-A- 0 462 031
- WO-A-91/15481
- WO-A-95/19334
- TETRAHEDRON LETTERS, Bd. 36, Nr. 7, 1995, OXFORD GB, Seiten 1133-1136, XP002039616 C. MALANGA ET AL.: "Nickel Mediated Flash Isomerization: a New Approach to 1,4-But-1-ene Diol Derivatives" in der Anmeldung erwähnt
- JOURNAL OF THE AMERICAN CHEMICAL SOCIETY, Bd. 89, Nr. 23, 8.November 1967, DC US, Seiten 5826-5831, XP002039617 T. OKUYAMA ET AL.: "Structure and Reactivity of alpha,beta-Unsaturated Ethers. The Acid-Catalyzed Hydrolysis of Alkenyl Alkyl Ethers" in der Anmeldung erwähnt

## Beschreibung

Die vorliegende Erfindung betrifft ein Verfahren zur Herstellung von Tetrahydrofuran durch Umsetzung von Dialkoxybutenen mit Wasser und Wasserstoff in Gegenwart von Katalysatoren oder Katalysatorkombinationen, die sowohl hydrieren können als auch saure oder basische Zentren aufweisen.

Tetrahydrofuran wird in großem Umfang technische durch Cyclisierung von Butandiol-1,4 (Weissermel, Arpe "Industrielle Organische Chem.", 4. Auflage, VCH Verlagsgesellschaft Weinheim, 1994, S. 111) erhalten. Eine weitere Herstellungsmöglichkeit besteht in der Hydrierung von Dihydrofuran (EP-A 524 216).

Ausgangsmaterialien für die Vorprodukte sind dabei Acetylen, Propylen bzw. Propylenoxid, Maleinsäureanhydrid oder Butadien. Wegen der sehr guten Verfügbarkeit von Butadien werden nun bevorzugt neue Verfahren gesucht, die von Butadien ausgehen und es gestatten, Tetrahydrofuran in einfacherer Weise und kostengünstiger herzustellen, wobei insbesondere die Zahl der Reaktionsstufen vermindert werden soll.

Auf dem Ausgangsmaterial Butadien basierende Zwischenprodukte sind Dialkoxybutene, die als 1,4-Dialkoxy-2-butene (in cis- und trans-Form) und als 1,4-Dialkoxy-1-butene vorliegen können.

Sie lassen sich durch die allgemeine Formeln

RO ― CH₂― CH = CH ― CH₂ ― OR I

bzw.

RO ― CH₂― CH₂― CH = CH ― OR II,

beschreiben, wobei R für gleiche oder verschiedene C₁- bis C₁₅-Alkyl- oder Cycloalkyl-, C₆- bis C₁₂-Aryl- oder C₇- bis C₁₅-Aralkylreste steht.

Die 1,4-Butendioldiether der Formeln I und II lassen sich auf verschiedene Weise herstellen, z.B. durch Umsetzung von Dibrombuten mit zwei Äquivalenten Alkoholen, durch oxidative Anlagerungen von Alkoholen an Butadien (SU-A 1 046 238 oder EP-A 462 031) oder durch Addition von Alkoholen an Vinyloxiran (WO-A 8 902 883).

Eine weitere Methode, die ebenfalls Gegenstand dieser Erfindung ist, besteht in der Anlagerung von Alkoholen an Butadien unter Bildung von Monoalkoxybutenen gemäß WO 95/19334 und deren Metathese zu Dialkoxybut-2-enen und 2-Buten.

Es wurde nun gefunden, daß man Tetrahydrofuran in wenigen Stufen ausgehend von Butadien in guter Ausbeute herstellen kann, wenn man 1,4-Butendioldiether der Formeln I und/oder II

RO ― CH₂― CH = CH ― CH₂ ― OR I

RO ― CH₂― CH₂ ― CH = CH ― OR II,

in der die Reste R gleich oder verschieden sein können und für C₁-C₁₅-Alkyl- oder Cycloalkylreste, für C₆-C₁₂-Arylreste oder für C₇-C₁₅-Aralkylreste stehen, mit Wasser und Wasserstoff bei Temperaturen von 20 bis 300°C und einem Druck von 1 bis 300 bar in Gegenwart von Katalysatoren oder Katalysatorkombinationen umsetzt, die sowohl Komponenten enthalten, die hydrieren können als auch saure oder basische Zentren aufweisen.

Es wird angenommen, daß die erfindungsgemäße Umsetzung über die im folgenden Schema wiedergegebenen Einzelschritte verläuft: 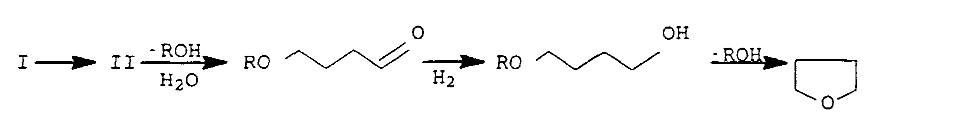 wobei der abgespaltene Alkohol zur Herstellung der Ausgangsverbindungen I und II zurückgeführt werden kann.

Dabei sind für die angenommenen einzelnen Schritte in der Literatur folgende Angaben vorhanden:

Die Isomerisierung von Dialkoxybut-2-enen zu den entsprechenden Dialkoxybut-1-enen ist noch nicht beschrieben. Als ähnlich anzusehen ist allerdings die Isomerisierung des Bistrimethylsilylbut-2-enethers zu Bistrimethylsilylbut-1-enethers (C. Malanga et al. Tetrahedron Lett. 36, 1133-1136 (1995) mit Nickelhydriden.

Auch die hydrolytische Spaltung von Dialkoxybut-1-enen zum entsprechenden Alkohol und Aldehydether ist nicht bekannt, es finden sich in der Literatur nur Beispiele zur säurekatalysierten Hydrolyse von einfachen Enolethern (z.B. T. Okuyama et al., J.Am.Chem. Soc. 89, 5826-5831 (1967)).

Die Hydrierung der Aldehydether mit Raney-Nickel als Katalysator (als Alkoxyrest werden ausschließlich in alpha-Position alkylierte Verbindungen verwendet) zu den entsprechenden 4-Alkoxybutanolen ist ebenso wie die anschließende Cyclisierung an sauren Katalysatoren zu THF in EP 18 164 B1 beschrieben. Allerdings wird dabei die Etherkomponente nicht nur als Alkohol abgespalten, sondern auch als entsprechend dehydratisiertes Produkt, d.h. als Olefin, welches sich in einem aus wirtschaftlichen Gründen bevorzugten Kreisprozeß nur umständlich wieder einsetzen läßt. Entsteht das Olefin, ist zudem nicht THF das Primärprodukt, sondern 1,4-Butandiol, welches erst nach Wasserabspaltung THF bildet.

Im Hinblick auf den vorgenannten Stand der Technik war es überraschend, daß es möglich ist, aus Dialkoxybutenen der Formeln I bzw. II ein- oder maximal zweistufig THF und gleichzeitig die Alkoholkomponente (damit mit der Möglichkeit der Rückführung und der Ausbildung eines Kreisprozesses) mit hoher Selektivität zu gewinnen.

Die Reaktion kann ein- oder zweistufig erfolgen.

Bei der einstufigen Variante werden die Verbindungen der Formeln I und/oder II in der Gas- oder Flüssigphase in Gegenwart von Wasser und Wasserstoff sowie eines Katalysators, der zu hydrieren vermag und der entweder Brönsted- und/oder Lewis-Säure- oder Basenzentren besitzt, oder dem ein entsprechender Brönsted- und/oder Lewis-saurer oder basischer Katalysator zugesetzt wurde, zu THF und Alkohol umgesetzt.

Die Reste R in Edukt und Zwischenprodukten können verschieden, bevorzugt aber gleich sein. Bevorzugt werden solche Reste verwendet, die nach Abspaltung einen primären Alkohol ergeben.

Bei der zweistufigen Variante werden Verbindungen der Formeln I und/oder II in Gegenwart von Wasser und Wasserstoff sowie eines Hydrierkatalysators zu 1,4-Butandiolmonoether umgesetzt, der dann, gegebenenfalls nach Zwischenreinigung an einem sauren oder basischen Katalysator zu THF und Alkohol umgesetzt wird.

Das ein- oder zweistufige Verfahren kann diskontinuierlich oder bevorzugt kontinuierlich durchgeführt werden.

Die Beschreibung der folgenden Verfahrensmerkmale gilt sowohl für die einstufige Arbeitsweise als auch die erste Stufe der zweistufigen Variante:

Das Molverhältnis Wasser zu 1,4-Butendioldiethern der Formeln I und/oder II beträgt zwischen 100:1, bevorzugt 50:1, besonders bevorzugt 10:1.

Der Reaktionsdruck - im wesentlichen durch Wasserstoff bedingt - beträgt 1 bis 300 bar, bevorzugt 1 bis 200 bar, besonders bevorzugt 1 bis 100 bar, die Reaktionstemperaturen liegen im Bereich von 20 bis 300°C, bevorzugt 40 bis 270°C, besonders bevorzugt 80 bis 200°C.

Erfindungsgemäß werden vor allem solche Katalysatoren verwendet, die Ketone oder Aldehyde katalytisch mit Wasserstoff zu Alkoholen zu hydrieren vermögen. Sie enthalten in der Regel eines oder mehrere Elemente der I., II., VI.-VIII. Nebengruppe oder III.-V. Hauptgruppe des Periodensystems der Elemente bzw. deren Verbindungen. Sie können homogen gelöst (Beispiele in H. Kropf, Methoden der organischen Chemie (Houben-Weyl), Band IV/1c, Georg Thieme Verlag Stuttgart, 1980, S. 45-67), oder als heterogene Katalysatoren vorliegen.

Bevorzugte Homogenkatalysatoren sind z.B. Komplexe des Rhodiums, Rutheniums, Iridiums, Palladiums, Platins und Kobalts mit Phosphin- oder Phosphit-Liganden, deren Herstellung z.B. in CA-A 7 276 41, H. Brunner in Hartley: The chemistry of the metalcarbon bond; Vol. 5, S. 110-124, John Wiley u. Sons, New York 1998 sowie Töth et al., Inorg. Chim. Acta 42, 153 (1980) und in der dort zitierten Literatur beschrieben wird. Ferner sind in WO 95-19 334 geeignete Metallkomplexe beschrieben.

Besonders bevorzugt sind Ru-Komplexe. Beispielhaft seien HRuCl(CO)(TPP)₃ und H₂Ru(CO)(TPP)₃ (TPP = Triphenylphosphin) genannt.

Die heterogenen Katalysatoren können sowohl fest angeordnet, als auch mobil, wie z.B. in einem Wirbelbettreaktor, oder in Suspension eingesetzt werden. Beispiele hierfür sind z.B. in Houben-Weyl, Methoden der Organischen Chemie, Band IV/1c, S. 16 bis 26 beschrieben.

Von diesen Hydrierkatalysatoren sind solche bevorzugt, die ein oder mehrere Elemente der Gruppe Ib, IIb, VIb, VIIb und VIIIb insbesondere Kupfer, Chrom, Rhenium, Kobalt, Rhodium, Nickel, Palladium, Ruthenium, Eisen und Platin oder deren Verbindungen enthalten.

Die im erfindungsgemäßen Verfahren eingesetzten Katalysatoren können z.B. sogenannte Fällungskatalysatoren sein. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder Carbonat-Lösungen, z.B. als schwerlösliche Hydroxide, Oxidhydrate, basische Salze oder Carbonate ausfällt, die erhaltenen Niederschläge anschließend trocknet und diese dann durch Calcinierung bei im allgemeinen 300 bis 700°C, insbesondere 400 bis 600°C in die entsprechenden Oxide, Mischoxide und/oder gemischtvalentigen Oxide umwandelt, welche durch eine Behandlung mit Wasserstoff oder mit Wasserstoff enthaltenden Gasen bei in der Regel 50 bis 700°C, insbesondere 100 bis 400°C zu den betreffenden Metallen und/oder oxidischen Verbindungen niederer Oxidationsstufe reduziert und in die eigentliche, katalytisch aktive Form überführt werden. Dabei wird. in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden. Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten. Außer den oben genannten Fällungskatalysatoren, welche außer den katalytisch aktiven Komponenten noch zusätzlich ein Trägermaterial enthalten, eignen sich für das erfindungsgemäße Verfahren im allgemeinen solche Trägermaterialien, bei denen die katalytisch-hydrierend wirkende Komponenten z.B. durch Imprägnierung auf ein Trägermaterial aufgebracht worden sind.

Die Art der Aufbringung der katalytisch aktiven Metalle auf den Träger ist in der Regel nicht kritisch und kann auf verschiedenerlei Art und Weise bewerkstelligt werden. Die katalytisch aktiven Metalle können auf diesen Tragermaterialien z.B. durch Tränkung mit Lösungen oder Suspensionen der Salze oder Oxide der betreffenden Elemente, Trocknung und anschließender Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, z.B. mit Nitraten oder thermisch leicht zersetzbaren Komplexverbindungen, z.B. Carbonyl- oder Hydrido-Komplexen der katalytisch aktiven Metalle, zu imprägnieren und den so getränkten Träger zwecks thermischer Zersetzung der adsorbierten Metallverbindungen auf Temperaturen von 300 bis 600°C zu erhitzen. Diese thermische Zersetzung wird vorzugsweise unter einer Schutzgasatmosphäre vorgenommen. Geeignete Schutzgase sind z.B. Stickstoff, Kohlendioxid, Wasserstoff oder die Edelgase.

Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Es versteht sich für den Fachmann von selbst, daß höhere Gehalte diese Trägerkatalysatoren an katalytisch aktiven Metallen zu höheren Raum-Zeit-Umsätzen führen können als niedrigere Gehalte. Im allgemeinen werden Trägerkatalysatoren verwendet, deren Gehalt an katalytisch aktiven Metallen 0,1 bis 90 Gew.-%, vorzugsweise 0,5 bis 40 Gew.-% bezogen auf den gesamten Katalysator beträgt. Da sich diese Gehaltsangaben auf den gesamten Katalysator inklusive Trägermaterial beziehen, die unterschiedliche Trägermaterialien jedoch sehr unterschiedliche spezifische Gewichte und spezifische Oberflächen haben, können diese Angaben aber auch unter- oder überschritten werden, ohne daß sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach dem Verfahren von DE-A 2 519 817, EP-A 1 477 219 und EP-A 285 420 auf den Träger aufgebracht werden. In den Katalysatoren gemäß den vorgenannten Schriften liegen die katalytisch aktiven Metalle als Legierung vor, die durch thermische Behandlung und/oder Reduktion der z.B. durch Tränkung mit einem Salz oder Komplex der zuvor genannten Metalle, erzeugt werden.

Sowohl die Aktivierung der Fällungskatalysatoren als auch der Trägerkatalysatoren kann auch in situ zu Beginn der Reaktion durch den anwesenden Wasserstoff erfolgen, bevorzugt werden diese Katalysatoren jedoch vor ihrer Verwendung separat aktiviert.

Als Trägermaterialien können im allgemeinen die Oxide des Aluminiums und Titans Zirkoniumdioxid, Siliciumdioxid, Tonerden, z.B. Montmorillonite, Silikate wie Magnesium- oder Aluminiumsilikate, Zeolithe wie ZSM-5 oder ZSM-10-Zeolithe, sowie Aktivkohle verwendet werden. Bevorzugte Trägermaterialien sind Aluminiumoxide, Titandioxide, Siliciumdioxid, Zirkoniumdioxid und Aktivkohle. Selbstverständlich können auch Mischungen verschiedener Trägermaterialien als Träger für im erfindungsgemäßen Verfahren anwendbaren Katalysatoren dienen.

Als für im erfindungsgemäßen Verfahren einsetzbaren Heterogenkatalysatoren seien die folgenden beispielhaft genannt:

Platin auf Aktivkohle, Palladium auf Aktivkohle, Palladium auf Aluminiumoxid, Kobalt auf Aktivkohle, Kobalt auf Siliciumdioxid, Kobalt auf Aluminiumoxid, Eisen auf Aktivkohle, Mangan auf Aktivkohle, Rhenium auf Aktivkohle, Rhenium auf Siliciumdioxid, Rhenium/Zinn auf Aktivkohlen, Rhenium /Palladium auf Aktivkohle, Kupfer auf Aktivkohle, Kupfer auf Siliciumdioxid, Kupfer auf Aluminiumoxid, Kupferchromit, Bariumkupferchromit sowie die Katalysatoren gemäß DE-A 3 932 332, US-A 3 449 445, EP-A 44 444, EP-A 147 219, DE-A 3 904 083, DE-A 2 321 101, WEP-A 415 202, DE-A 2 366 264 und EP-A 100 406.

Besonders bevorzugte Katalysatoren enthalten mindestens eines der Metalle Kupfer, Ruthenium oder Rhenium.

Brönsted- und/oder Lewissäuren oder Basen können für die Umsetzung in einer Gasphasenreaktion auf dem Katalysator aufgebracht, bei Reaktionen in der Flüssigphase auch homogen gelöst sein. Bevorzugt sind Brönsted- und/oder Lewissäuren. Bevorzugt eingesetzte Säuren oder Basen sind unten aufgeführt.

Wird die Reaktion zweistufig durchgeführt, so kann der Reaktionsaustrag aus der ersten Stufe, gegebenenfalls nach Katalysatorabtrennung, entweder direkt, oder vorteilhaft durch Destillation in die Bestandteile Alkohol, gegebenenfalls überschüssiges Wasser, und 4-Alkoxybutanol zerlegt werden, wobei letzteres zur THF-Freisetzung, entweder in der Gas- oder Flüssigphase, weiterverwendet wird.

Die Cyclisierung zu THF und Alkohol kann an basischen oder bevorzugt sauren, homogenen oder heterogenen Katalysatoren durchgeführt werden. Basische Katalysatoren sind z.B. Alkali- oder Erdalkalimetalloxide oder Hydroxide bzw. Trägermaterialien, die diese alkalischen Komponenten z.B. aufgetränkt oder aufgesprüht enthalten oder basische Ionentauscher.

Saure Katalysatoren sind z.B. Zeolithe in der H-Form, saure Ionentauscher, Heteropolysäuren, saure und supersaure Metalloxide, die gegebenenfalls mit Sulfat oder Phosphat dotiert wurden und anorganische oder organische Säuren.

Als Zeolithe eignen sich beispielsweise Vertreter aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe gehören auch die sogenannten "ultrastabilen" Zeolithe des Faujasittyps, d.h. dealuminierte Zeolithe.

Besonders vorteilhaft unter den Zeolithen sind solche mit Pentasilistruktur wie ZSM-5, ZSM-11 und ZBM-10. Diese haben als Grundbaustein einen aus SiO₂-Tetraedern aufgebauten Fünfring gemeinsam. Sie sind durch ein hohes SiO₂/Al₂O₃-Verhältnis gekennzeichnet sowie durch Porengrößen, die zwischen denen der Zeolithe vom Typ A und denen vom Typ X oder Y liegen.

Als saure Katalysatoren kommen gleichermaßen Heteropolysäuren in Betracht, z.B. anorganische Polysäuren, die im Gegensatz zu Isopolysäuren mindestens zwei verschiedene Zentralatome besitzen. Als Beispiele seinen Dodecawolframphosphorsaure H₃PW₁₂O₄₀·xH₂O, Dodecamolybdophosphorsäure H₃PMo₁₂O₄₀·xH₂O genannt. Prinzipiell können die in EP-A 158 229 genannten Katalysatoren und Katalysatorkombinationen eingesetzt werden.

Bevorzugte Heteropolysäuren sind Heteropolysauren von Molybdän oder Wolfram mit Phosphorsäure, Tellursäure, Selensäure, Arsensäure, Kieselsäure, insbesondere mit Phosphorsäure.

Die Protonen der Heteropolysäuren können teilweise durch Metallionen ersetzt sein, bevorzugt sind dabei die Alkali- und Erdalkalimetallionen.

Auch saure Ionentauscher z.B. vernetzte Polystyrole mit Sulfonsäuregruppen oder saure Metalloxide, beispielsweise SiO₂, Al₂O₃, ZrO₂, TiO₂, SnO₂, TiO₂ usw. oder Kombinationen einzelner Oxide kommen in Betracht. Die Oxide können zur Saurestarkeerhöhung auch mit Mineralsäuren wie z.B. Schwefelsäure behandelt werden.

Als Säuren sind auch Mineralsäuren, wie Schwefelsäure und Phosphorsäure sowie organische Säuren, wie z.B. Sulfonsäuren geeignet.

Falls die Umsetzung in 2 Stufen durchgeführt wird, wird bei der erfindungsgemäßen Umsetzung eine Reaktionstemperatur in der zweiten Stufe von 30 bis 300°C, bevorzugt 40 bis 280°C, besonders bevorzugt 60 bis 250°C eingehalten.

Der Reaktionsdruck kann je nach gewähltem System leichtes Vakuum oder leichter Überdruck sein. Im allgemeinen liegt er zwischen 0,1 und 10 bar, bevorzugt 0,5 bis 5 bar, besonders bevorzugt 0,8 bis 4 bar.

Die Reaktionsprodukte können durch einen Inertgasstrom, vorzugsweise aus homogenen Reaktionsansätzen, entfernt werden.

Die Reaktionsprodukte - THF und Alkohol - werden in an sich bekannter Weise getrennt, bevorzugt durch Destillation.

Eine weitere Möglichkeit das erfindungsgemäße Verfahren durchzuführen ist eine Kombination aus ein- bzw. zweistufiger Variante. Dabei kann das unter den Bedingungen der einstufigen Verfahrensführung nicht vollständig zu THF und Alkohol umgesetzte 4-Alkoxybutanol unter den erfindungsgemäßen Bedingungen der zweiten Stufe vollständig umgesetzt werden.

In einer weiteren Ausführungsform des erfindungsgemäßen Verfahren werden
a) But-2-en-1-olether der Formel III

   CH₃― CH = CH ― CH₂ ― OR III,

   in denen R für einen C₁-C₁₅-Alkyl- oder Cycloalkylrest, für einen C₆-C₁₂-Arylrest oder für einen C₇-C₁₅-Aralkylrest steht, in Gegenwart eines Metathesekatalysator zu Buten und den entsprechenden 1,4-Butendioldiethern der Formel I umgesetzt und anschließend
b) die 1,4-Butendioldiether der Formel I allein oder in Kombination mit den 1,4-Butendiolethern der Formel II zu Tetrahydrofuran umgesetzt.

Die Reaktion verläuft nach dem folgenden Schema, wobei 1,4-Dialkoxybutenen aus 1-Alkoxybutenen unter Abspaltung von 2-Buten in Gegenwart eines Katalysators gebildet wird

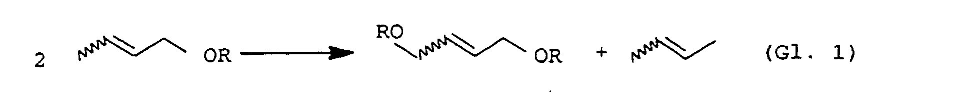

Erfindungsgemäße zu verwendende Metathesekatalysatoren sind solche, die in der Lage sind, Olefine gemäß Gl. 2 zu metathetisieren.

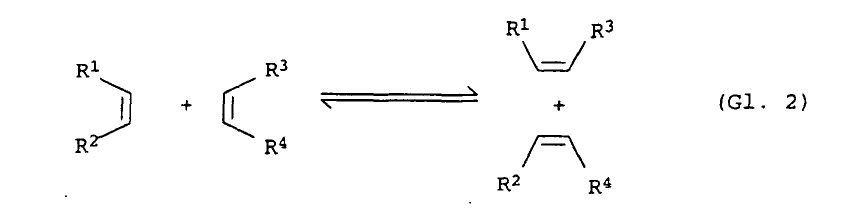

Als Metathesekatalysatoren kommen homogene und heterogene Verbindungen der Übergangsmetalle, insbesondere der IV., VI., VII. und VIII. Nebengruppe des Periodensystems der Elemente sowie homogene und heterogene Katalysatorsysteme, in denen diese Verbindungen enthalten sind, in Betracht. Beispiele für solche Katalysatoren sind in der Literatur beschrieben (z.B. G.W. Parshall, S.D. Ittel, Homogeneous Catalysis, 2. Auflage, 1992, Wiley, S. 217ff; R.L. Banks, Catalysis, Bd. S. 100ff.; R.H. Grubbs, Progress in Inorg. Chem., Bd. 24, S. 1ff).

Bevorzugte Katalysatoren sind Rutheniumverbindungen der allgemeinen Zusammensetzung RuX₂(=CHR) (PR'₃)₂ wie sie von Grubbs in WO 93/20111 beschrieben werden, wobei X Halogen, R Wasserstoff, Alkyl oder Aryl und R' Alkyl bedeutet. Ferner kommen bevorzugt Gemische der Verbindungen der Formel [Ru(η⁶-Aren)X₂]₂/(PR₃)/N₂CHR', deren Eignung als Metathesekatalysator von Noels in J. Chem. Soc., Chem. Commun.; 1995, S. 1127ff. beschrieben ist, in Betracht, wobei Aren z.B. Benzol, Mesitylen oder Cymol, X Halogen, R Alkyl und R' Wasserstoff, Alkyl, Aryl oder Trimethylsilyl bedeutet.

Besonders bevorzugt ist der Komplex RuCl₂(=CHPh)(PCy₃)₂ (Cy = Cyclohexyl) sowie ein Katalysatorsystem basierend auf den kommerziell verfügbaren Verbindungen [Ru(η⁶-p-Cymol)Cl₂]₂/PCy₃/N₂CHSiMe₃. Anstelle der Komponenten Ru(η⁶-p-Cymol)Cl₂]₂ und PCy₃ kann auch deren Reaktionsprodukt, der Komplex [Ru(η⁶-p-Cymol) (PCy₃)Cl₂], eingesetzt werden.

In US 5 342 985 (DE 39 40 196 A) und J. Chem. Soc., Chem. Commun. 1979, S. 330-331 wird die Synthese von 1,4-Dialkoxybutenen aus den entsprechenden Allylethern unter Abspaltung von Ethylen in Gegenwart heterogener Re-Verbindungen als Metathesekatalysatoren beschrieben.

Es ist jedoch aus der Literatur bekannt, daß Übergangsmetall-Verbindungen, insbesondere die des Rutheniums, in der Lage sind, ungesättigte Ether wie Allyl- und Butenylether gemäß Gl. 3 zu isomerisieren. In Gegenwart von Wasser findet dann eine Etherspaltung statt, woraus die entsprechenden Aldehyde resultieren.

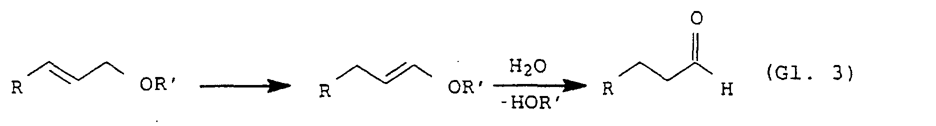

Deshalb war es überraschend, daß die gegenüber Metathesereaktionen (im Vergleich zu den Allylethern) prinzipiell reaktionsträgeren 1-Alkoxybut-2-ene in Gegenwart von Ru-Verbindungen nicht zu den 1-Alkoxy-1-butenen isomerisieren, sondern selektiv unter Abspaltung von 2-Buten 1,4-Dialkoxybutene bilden.

Da die Metathesereaktion eine technisch und gegenüber der Verwendung von Dibrombutenen auch umwelttechnisch günstige Alternative darstellt, ist die Verwendung der durch Metathese hergestellten Butendioldiether als Edukte für die erfindungsgemäße Tetrahydrofuranherstellung bevorzugt.

Das erfindungsgemäße Verfahren wird anhand nachfolgender Beispiele näher erläutert, jedoch in keiner Weise eingeschränkt. Die Analysen erfolgten über Gaschromatographie. Das in den Beispielen eingesetzte Dibutoxibuten wies eine Reinheit von ca. 98 % auf.

### Beispiel 1:

In einen 200 ml Rohrreaktor wurden 200 ml eines Kupfer/Aktivkohle-Katalysators (Cu-Gehalt berechnet als CuO ca. 10 % auf 4 mm Aktivkohle-Strängen; Kupfer als Ammoniakat aufgebracht) gefüllt und bei ca. 180°C im Wasserstoffstrom aktiviert. Danach wurden über zwei Zuläufe eine 10 gew.-%ige Lösung von Dibutoxybut-2-en in n-Butanol (11 g/h) und 11 g Wasser/h jeweils gasförmig im Wasserstoffstrom (40 l/h) bei 1 bar und 230°C über die Katalysatorzone geleitet. Der Reaktionsaustrag war zweiphasig. In Stundenabständen wurden Proben gezogen und gaschromatographisch analysiert. In der organischen Phase befanden sich (wasserfrei gerechnet) zwischen 95 und 98 % n-Butanol und ca. 0,8 % THF. In der Wasserphase befanden sich (wasserfrei gerechnet) 95 % n-Butanol und ca. 0,5 % THF.

### Beispiel 2:

Analog Beispiel 1 wurden 200 ml eines Rhenium/Aktivkohle-Katalysators (Re-Gehalt ca. 6 Gew.-% auf 4 mm Aktivkohle-Strängen, Re als Re₂O₇ aufgebracht) bei 300°C aktiviert. Danach wurden ca. 12 g/h Dibutoxybut-2-en und ca. 11 g Wasser/h bei 220°C und einem H₂-Trägergasstrom von 25 1/h über den Katalysator geleitet. Es fanden sich im Austrag 59 % Butanol und 30 % THF.

### Beispiel 3: (zweistufige Herstellung)

a) In einem 72 ml Metallautoklaven wurden 5 g Dibutoxybut-2-en, 5 g Wasser, 0,1 g HRuCl(CO)(TPP)₃ und 0,05 g TPP (Triphenylphosphin) eingefüllt und 50 bar Wasserstoff aufgepreßt. Danach wurde der Autoklav unter Rühren auf 150°C aufgeheizt und nach 2 Stunden abgekühlt. Der Restdruck betrug 40 bar. Der zweiphasige Reaktionsaustrag wurde für analytische Zwecke mit 13 g Methanol homogenisiert. Es wurden ca. 80 % 4-Butoxibutanol und ca. 17 % Butanol gefunden.
b) 10 g pulverisiertes Al₂O₃ und 18 g 4-Butoxybutanol wurden in den Sumpf einer Destillationsapparatur vorgelegt und auf 175°C aufgeheizt. Bei dieser Temperatur destillierten die Reaktionsprodukte über Kopf ab. Es wurde kontinuierlich immer so viel frisches 4-Butoxybutanol ergänzt, wie Produkte abdestillierten. Im Destillat fanden sich ca. 3 % Edukt, 52 % Butanol und 45 % THF.

### Beispiel 4: (Herstellung von Dialkoxybuten durch Metathese)

In einem Glasgefäß wurden unter Argonatmosphäre 41 mg Ru(Cl₂(=CHPh)(PCy₃)₂ (Cy = Cyclohexyl) mit 8,0 g 1-Butoxybut-2-en versetzt und bei Raumtemperatur gerührt. Das im Reaktionsverlauf entstehende Buten konnte über einen Blasenzähler entweichen. Nach 12 h wurde das Reaktionsgemisch gaschromatographisch (GC/MS-Kopplung) analysiert. Neben der Ausgangsverbindung 1-Butoxybut-2-en (51,6 und 18,6 Fl.-%, E-, Z-Isomer) und Dibutylether (4,2 Fl.-%) wurden 1,4-Dibutoxybut-2-en (4,78 und 17,1 Fl.-%, E-, Z-Isomer) und 2-Buten (2,9 Fl.-%) nachgewiesen. Als Nebenkomponente wurde 1,4-Dibutoxybut-1-en (0,7 Fl.-%) identifiziert.

### Beispiel 5:

Man verfuhr wie in Beispiel 4 beschrieben, jedoch unter Verwendung von [Ru(η⁶-p-Cymol)(PCy₃)Cl₂]/N₂CHSiMe₃ als Katalysator. Nach 12 h bei 60°C wird gaschromatographisch 1,4-Dibutoxybuten (1,9 und 3,0 Fl.-%) nachgewiesen.

## Patentansprüche

1. Verfahren zur Herstellung von Tetrahydrofuran, **dadurch gekennzeichnet, daß** man 1,4-Butendioldiether der Formeln I und/oder II
RO ― CH₂― CH = CH ― CH₂ ― OR I
RO ― CH₂― CH₂ ― CH = CH ― OR II,
in der die Reste R gleich oder verschieden sein können und für C₁-C₁₅-Alkyl- oder Cycloalkylreste, für C₆-C₁₂-Arylreste oder für C₇-C₁₅-Aralkylreste stehen, mit Wasser und Wasserstoff bei Temperaturen von 20 bis 300°C und einem Druck von 1 bis 300 bar in Gegenwart von Katalysatoren oder Katalysatorkombinationen umsetzt, die sowohl Komponenten enthalten, die hydrieren können als auch saure oder basische Zentren aufweisen.

2. Verfahren gemäß Anspruch 1, **dadurch gekennzeichnet, daß** man Katalysatoren verwendet, die in der Hydrierkomponente eines oder mehrere Elemente der I., II. oder VI.-VIII. Nebengruppe oder der III.-V. Hauptgruppe des Periodensystems der Elemente oder deren Verbindungen enthalten.

3. Verfahren gemäß Anspruch 1, **dadurch gekennzeichnet, daß** man Katalysatoren verwendet, die in der Hydrierkomponente eines oder mehrere der Elemente Rhenium, Kupfer oder Ruthenium oder deren Verbindungen enthalten.

4. Verfahren gemäß Anspruch 1, **dadurch gekennzeichnet, daß** man die Reaktion in 2 Stufen durchführt, indem man
a) in einer ersten Stufe die Butendioldiether der Formeln I und/oder II mit Wasser und Wasserstoff in Gegenwart eines Hydrierkatalysators zu dem entsprechenden 1,4-Butandiolmonoether umsetzt und diesen
b) in Gegenwart von sauren oder basischen Katalysatoren bei 40 bis 280°C zu Tetrahydrofuran cyclisiert.

5. Verfahren nach Anspruch 1, **dadurch gekennzeichnet, daß** man
a) But-2-en-1-olether der Formel III
CH₃― CH = CH ― CH₂ ― OR III,
in denen R für einen C₁-C₁₅-Alkyl- oder Cycloalkylrest, für einen C₆-C₁₂-Arylrest oder für einen C₇-C₁₅-Aralkylrest steht in Gegenwart eines Metathesekatalysator zu Buten und den entsprechenden 1,4-Butendioldiethern der Formel I umsetzt und anschließend
b) die 1,4-Butendioldiether der Formel I allein oder in Kombination mit den 1,4-Butendiolethern der Formel II zu Tetrahydrofuran umsetzt.

6. Verfahren gemäß Anspruch 5, **dadurch gekennzeichnet, daß** man Metathesekatalysatoren verwendet, die Ruthenium- oder Rutheniumverbindungen enthalten.

7. Verfahren gemäß Anspruch 5, **dadurch gekennzeichnet, daß** man als Metathesekatalysatoren Verbindungen des Typs RuCl₂(=CHR)(PR'₃)₂ verwendet, wobei R Wasserstoff, Alkyl oder Aryl und R' Alkyl bedeutet.

8. Verfahren gemäß Anspruch 5, **dadurch gekennzeichnet, daß** man als Metathesekatalysatoren Verbindungen der Formel [RuX₂(η⁶-p-Cymol)PCy₃]/N₂CHR' verwendet, in der X Halogen, Cyclohexyl- und R' Wasserstoff, Alkyl, Aryl oder Trimethylsilyl bedeutet.

9. Verfahren gemäß Anspruch 5, **dadurch gekennzeichnet, daß** man 1-Butoxybut-2-en in Gegenwart eines Metathesekatalysators zu 2-Buten und 1,4-Dibutoxybuten umsetzt.

## Claims

1. A process for preparing tetrahydrofuran, which comprises reacting 1,4-butenediol diethers of the formulae I and/or II
RO ― CH₂― CH = CH ― CH₂ ― OR I
RO ― CH₂― CH₂ ― CH = CH ― OR II,
where the R radicals can be identical or different and are C₁-C₁₅-alkyl or cycloalkyl radicals, C₆-C₁₂-aryl radicals or C₇-C₁₅-aralkyl radicals, with water and hydrogen at from 20 to 300°C under from 1 to 300 bar in the presence of catalysts or catalyst combinations which comprise components which are both capable of hydrogenation and have acidic or basic centers.

2. A process as claimed in claim 1, wherein catalysts which comprise in the hydrogenation component one or more elements of subgroup I, II or VI-VIII or of main group III-V of the periodic table of the elements, or their compounds are used.

3. A process as claimed in claim 1, wherein catalyst which comprise in the hydrogenation component one or more of the elements rhenium, copper or ruthenium or their compounds are used.

4. A process as claimed in claim 1, wherein the reaction is carried out in two stages by
a) in a first stage reacting the butenediol diethers of the formulae I and/or II with water and hydrogen in the presence of a hydrogenation catalyst to give the corresponding 1,4-butanediol monoethers, and
b) cyclizing the latter in the presence of acidic or basic catalysts at 40 to 280°C to tetrahydrofuran.

5. A process as claimed in claim 1, which comprises
a) reacting 2-butenol ethers of the formula III
CH₃― CH = CH ― CH₂ ― OR III,
where R is a C₁-C₁₅-alkyl or cycloalkyl radical, a C₆-C₁₂-aryl radical or a C₇-C₁₅-aralkyl radical, in the presence of a metathesis catalyst to give butene and the corresponding 1,4-butenediol diethers of the formula I, and then
b) reacting the 1,4-butenediol diethers of the formula I alone or in combination with the 1,4-butenediol ethers of the formula II to give tetrahydrofuran.

6. A process as claimed in claim 5, wherein metathesis catalysts which comprise ruthenium or ruthenium compounds are used.

7. A process as claimed in claim 5, wherein compounds of the type of RuCl₂(=CHR)(PR'₃)₂ where R is hydrogen, alkyl or aryl and R' is alkyl are used as metathesis catalysts.

8. A process as claimed in claim 5, wherein compounds of the formula [RuX₂(η⁶-p-cymene)PCy₃]/N₂CHR' where X is halogen, Cy is cyclohexyl and R' is hydrogen, alkyl, aryl or trimethylsilyl are used as metathesis catalysts.

9. A process as claimed in claim 5, wherein 1-butoxy-2-butene is converted in the presence of a metathesis catalyst into 2-butene and 1,4-dibutoxybutene.

## Revendications

1. Procédé de préparation du tétrahydrofuranne, **caractérisé en ce que** l'on convertit des 1,4-butènedioldiéthers de formule I et/ou II
RO - CH₂ - CH = CH - CH₂ - OR I
RO - CH₂ - CH₂ - CH = CH - OR II
dans lesquelles les radicaux R peuvent être semblables ou différents et désignent des groupes alkyles ou cycloalkyles en C₁ à C₁₅, des radicaux aryles en C₆ à C₁₂ ou des radicaux aralkyles en C₇ à C₁₅, avec de l'eau et de l'hydrogène à des températures comprises entre 20 et 300°C et sous une pression comprise entre 1 et 300 bars en présence de catalyseurs ou de combinaisons de catalyseurs qui comportent aussi bien des composants hydrogénants que des centres acides ou basiques.

2. Procédé selon la revendication 1, **caractérisé en ce que** l'on utilise des catalyseurs qui contiennent dans les composants hydrogénants un ou plusieurs éléments des groupes de transition I, II ou III à VIII ou des groupes principaux III à V du système périodique des éléments ou des composés de ceux-ci.

3. Procédé selon la revendication 1, **caractérisé en ce que** l'on utilise des catalyseurs qui contiennent dans les composants hydrogénants un ou plusieurs éléments des composés de rhénium, cuivre ou ruthénium ou des composés de ceux-ci.

4. Procédé selon la revendication 1, **caractérisé en ce que** l'on réalise la réaction en 2 étapes,
a) en convertissant dans une première étape les butènedioldiéthers de formule I et/ou II en le 1,4-butanediolmonoéther correspondant avec de l'eau et de l'hydrogène en présence d'un catalyseur hydrogénant et
b) en rendant celui-ci cyclique et en le transformant en tétrahydrofuranne en présence de catalyseurs acides ou basiques entre 40 et 280°C.

5. Procédé selon la revendication 1, **caractérisé en ce que**
a) l'on convertit des but-2-èn-1-oléthers de formule III
CH₃ - CH = CH - CH₂ - OR III
dans laquelle R représente un radical alkyle ou cycloalkyle en C₁ à C₁₅, un radical aryle en C₆ à C₁₇ ou un radical aralkyle en C₇ à C₁₅, en butène et en les 1,4-butènedioldiéthers de formule I correspondants en présence d'un catalyseur de métathèse puis
b) l'on convertit les 1,4-butènedioldiéthers de formule I seuls ou en combinaison avec les 1,4-butènediols de formule II en tétrahydrofuranne.

6. Procédé selon la revendication 5, **caractérisé en ce que** l'on utilise des catalyseurs de métathèse qui contiennent du ruthénium ou des composés de ruthénium.

7. Procédé selon la revendication 5, **caractérisé en ce que** l'on utilise comme catalyseurs de métathèse des composés du type RuCl₂(=CHR)(PR'₃)₂, R représentant un atome d'hydrogène, un alkyle ou un aryle et R' un alkyle.

8. Procédé selon la revendication 5, **caractérisé en ce que** l'on utilise comme catalyseurs de métathèse des composés de formule [RuX₂(η⁶-p-Cymol)PCy₃]/N₂CHR', dans laquelle X représente un halogène, un cyclohexyle et R' un atome d'hydrogène, un alkyle, un aryle ou un triméthylsilyle.

9. Procédé selon la revendication 5, **caractérisé en ce que** l'on convertit du 1-butoxybut-2-ène en 2-butène et en 1,4-dibutoxybutène en présence d'un catalyseur de métathèse.
